# EUROPEAN PATENT APPLICATION

(11) **EP 1 889 570 A2**
(43) Date of publication of application: **20.02.2008**
(21) Application number: 07075999.8
(22) Date of filing: 26.02.2007
(51) Int. Cl.: A61B 5/00, G01N 27/416, G01N 33/487

(54) **Usability methods of wirelessly calibrating an analyte measurement meter and corresponding system**

(30) Priority: 24.02.2006 US 776767 P
(62) Divisional of application: 07250793.2
(71) Applicant: Lifescan Scotland Ltd, Inverness IV2 3ED (GB)
(72) Inventor: Robinson, Grenville, Rosemarkie IV10 8UL (GB)
(74) Representative: Tunstall, Christopher Stephen

(57) **Abstract**

A measuring system including a meter, a container of test sensors, and a clip having two receiving portions for receiving the meter and the container and holding them in fixed relation to one another.

## Description

### BACKGROUND OF THE INVENTION

### 1. Field of the Invention

The present invention relates, in general, to a device used for monitoring an analyte, such as a blood glucose measurement meter, and to means and methods of conveying information to such a device.

### 2. Problem to be Solved

Systems for measuring the concentration of a specific analyte, indicator, or marker (hereinafter "analyte") from a sample of body fluid such as, for example, whole blood, plasma or interstitial fluid are commonly known and documented. For many individuals who suffer from a particular disease, such as diabetes, measurement of their analytes such as blood glucose is a necessary part of daily life. Such patients are advised by their health care professional to monitor their blood sugar levels regularly each day, typically ranging between two and six tests per day. To do this, patients rely on measurement systems are commercially available. These systems typically include a meter, disposable test sensors and lancets, such as those sold under the One Touch® Ultra brand from Lifescan, Inc., Milpitas, California, USA.

In order to more fully describe the problem to be solved, reference will be made to the specific disease, diabetes, and to diabetics. Reference to this disease is intended only to help clarify understanding. It is not intended to limit understanding or use of any information in this document to that specific disease.

As stated above, diabetics typically use a system that employs a disposable test sensor (also known as test strips) in a blood glucose meter that can be given them by their healthcare professional (HCP) or purchased. A diabetic will insert a test strip into a meter and apply a drop of his blood to the test area. In an electrochemical device, the test area will typically include a chemical system to change the glucose molecules in the drop of blood into ionic derivatives. When a voltage or current is applied across the test area, a resultant voltage or current can measured that is directly proportional to the amount of glucose in the drop of blood. This resultant voltage or current can then be, through an algorithm in the meter, used to calculate the amount of glucose in that sample and, hence, in the diabetic patient.

When using this type of test strip and meter, it is often necessary to make compensations for such things as temperature at the time of measurement as these factors will affect the accuracy and precision of the meter. Similarly, the process of manufacturing test strips may often result in a degree of variability between lots or batches. This variability is due to many factors among which are lot-to-lot variations in the test strip components during manufacture. Thus, during manufacture, each batch of test sensors is assigned a calibration code which, when input into the meter, will compensate for this variability so that all test strips will measure the same amount of blood glucose in a given sample with the same degree of accuracy and precision, regardless of the lot. This calibration code is used in the algorithm of the meter to compensate for such lot-to-lot manufacturing variability.

Each time a user purchases a new vial (taken herein to include alternative terms such as cartridge, dispenser or other container) of test sensors it will have assigned to it one of a number of different calibration codes. It is possible for the new vial to have the same calibration code as the previous vial used; however, it is likely that it will be different. Most meters currently available require the user to read the calibration code assigned to the new vial and manually enter this code into the meter prior to use.

Calibrating the meter each time a new vial of sensors is started, or indeed each time the user wishes to perform a test, can be inconvenient, and potentially life-threatening, due to the number of steps involved and the time consuming process of having to check the calibration code printed on the label of the vial. It is potentially inconvenient for the user to perform this step, particularly if the code required is printed on packaging that potentially could have been discarded or if the user is in a hurry, for example, experiencing a period of hypoglycemia, in which case their thought processes could be blurred.

Looking for small print on a label can be problematic for many diabetics, too, as diminished eyesight is often a resultant complication of the disease. Many users may forget to enter the calibration code or they may decide not to enter it if they do not understand its significance. Obtaining a result, such as a blood glucose concentration from a meter and strip system that is not properly calibrated, may be incorrect and potentially harmful to the user. An incorrect result may cause them to take inappropriate action. For reasons including those described herein it is desirable for the measurement system to include automatic calibration, and for the number of steps required by the user in order to perform a measurement to be kept to a minimum.

### SUMMARY OF THE INVENTION

The present invention overcomes many of the issues described above. A method of calibrating test sensors is disclosed herein, that requires minimum user intervention and removes many of the extra steps currently performed by users of conventional meters.

Provided is an analyte measuring system including a meter and a vial of test sensors, whereby calibration information specific to a particular vial of test sensors is transmitted wirelessly from a radio frequency identification (RF or RFID) tag incorporated within the vial, at a predefined time or within a predefined period, to a reader housed within the meter. The process of calibration may or may not be completely automated. For example, the user may be prompted to press a dedicated button, or bring the vial into contact with the meter, or somehow confirm that the calibration code transmitted from the vial to a receiver within the meter is correct. Alternatively the process of calibration may be completely automated requiring no action or input by the user, thereby simplifying the process of performing a test and ultimately reducing the time taken.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:

Figure 1 shows a flow diagram of the process steps involved in calibrating a conventional meter.

Figure 2 shows a simplified drawing of an example system for use with the present invention.

Figure 3 shows an example plot of an analyte measurement reaction versus time.

Figure 4 shows a process flow diagram outlining several different polling opportunities within a measurement cycle according to the present invention.

Figure 4a shows a simplified process flow of the main steps involved in polling option 1 of Figure 4.

Figure 4b shows a simplified process flow of the main steps involved in polling option 2 of Figure 4.

Figure 4c shows a simplified process flow of the main steps involved in polling option 3 of Figure 4.

Figure 4d shows a simplified process flow of the main steps involved in polling option 4 of Figure 4.

Figure 4e shows a simplified process flow of the main steps involved in polling option 5 of Figure 4.

Figure 4f shows a simplified process flow of the main steps involved in polling option 6 of Figure 4.

Figure 4g shows a simplified process flow of the main steps involved in polling option 7 of Figure 4.

Figure 4h shows a simplified process flow of the main steps involved in polling option 8 of Figure 4.

Figure 4i shows a simplified process flow of the main steps involved in polling option 9 of Figure 4.

Figure 5 shows a table of information that may be loaded from a RFID tag to a meter, or from a meter to the RFID tag in accordance with the present invention.

Figure 6 shows a first example embodiment of a system according to the present invention including a clip.

Figure 7 shows a flow diagram of the steps involved in calibrating the meter of Figure 6.

Figure 8 shows an example system according to a second embodiment of the present invention, including a dedicated calibration button.

Figure 9 shows a perspective view of the system of Figure 8, showing a vial contacting the meter and also an LED indicator.

Figure 10 shows a flow diagram of the steps involved in calibrating the system of Figures 8 and 9.

Figure 11 shows an example system according to a third embodiment of the present invention, including a micro-switch.

Figure 12 shows a perspective view of the system of Figure 11, showing a recess in the base of the meter and the location of a micro-switch.

Figure 13 shows a flow diagram of the steps involved in calibrating the system of Figures 11 and 12.

Figure 14 shows a further example embodiment of a system according to the present invention incorporating a cradle.

### DETAILED DESCRIPTION OF ILLUSTRATIVE EMBODIMENTS OF THE INVENTION

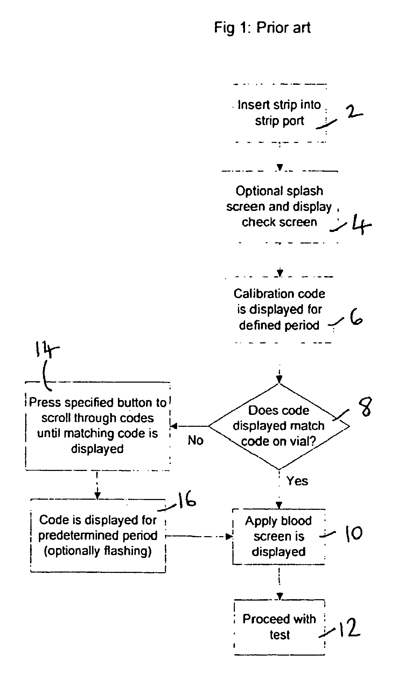

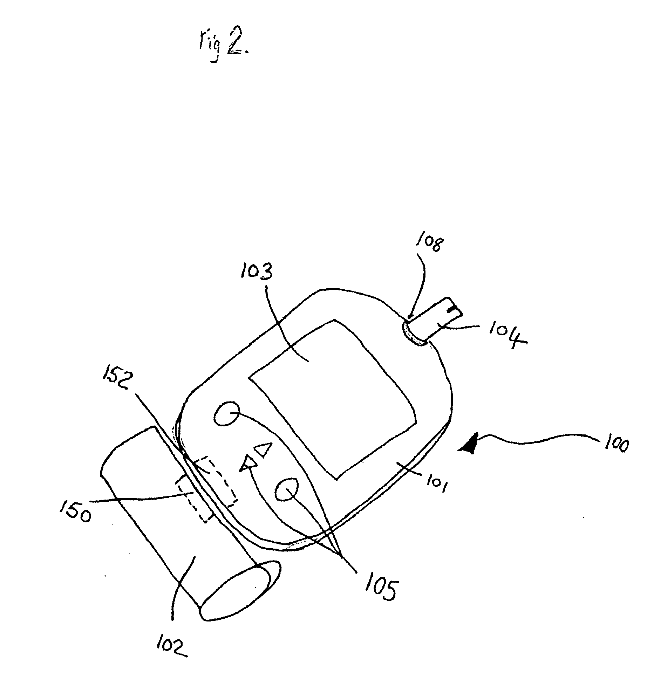

Looking at **Figures 1 and 2,** a system kit used to test a patient's blood glucose level typically consists of a meter 101, a lancet (not shown) and a plurality of disposable test sensors or strips 102, optionally contained within a re-closeable, moisture impermeable container such as a desiccated vial 104. **Figure 1** shows a flow diagram of process steps involved in calibrating a conventional analyte measuring meter, such as the type of meters used by diabetic patients for self-measurement of their blood glucose concentration. Calibrating the system, to account for any lot-to-lot variability in response of the test sensors to the analyte being measured caused by the manufacturing process, is important to achieve accurate and reliable results.

To perform a test, the user first removes a new test sensor from a vial and inserts it into the receiving strip port of the meter, step 2. For some meters, for example the OneTouch® Ultra from Lifescan Inc., Milpitas, California, USA, the action of inserting a strip into the receiving connector initiates automatic power-on of the meter. First, optionally a splash screen may be displayed followed by a screen check, step 4. Next, a calibration code is typically displayed for a predefined period of time, and optionally may flash, step 6. If the code displayed matches the calibration code printed on the vial, step 8, then the user can accept the code shown by the meter by either pressing a confirmation button or letting the display time out. The display will then show a prompt indicating that the system is ready to accept a sample, step 10. The user may then proceed to perform the test, step 12.

If however the code currently set within the meter does not match the code printed on the vial of test sensors, then the user presses a specific button on the meter to scroll through all the calibration code options until the correct number is reached, step 14. Once selected, the new code is displayed to the user for a predetermined period of time, e.g. 3 seconds, step 16, and optionally the number may flash. During this time, the user may confirm the new code by pressing a button or by letting the display time out.

**Figure 2** is a simplified schematic drawing of an example embodiment of an analyte monitoring system for use with the present invention. Monitoring system kit 100 includes a measurement meter 101, including a strip port 108, an internal RFID reader 152, display 103 and a set of buttons 105, a vial 102 or otherwise suitable container for holding blood glucose test sensors 104 including an RFID tag 150. It would be apparent to a person skilled in the art that containers or cartridges designed to house test sensors may take a form different from the vial shown herein. Optionally the container may be a vial, cartridge, cassette, dispenser, for example, and may be of any suitable shape, e.g., cylindrical, rectangular or disk-shaped. For the purpose of this application the term 'vial' will be used to encompass all types of container used for holding test sensors.

A measurement system, such as the simplified system of **Figure 2**, may be used for the routine determination of blood glucose by patients suffering from, for example, diabetes. For the purpose of this disclosure, the analyte of interest will be limited to blood glucose concentration, however it would be apparent to a person skilled in the art that monitoring meters or systems for the measurement of characteristics of other analytes or indicators may also incorporate the invention disclosed herein.

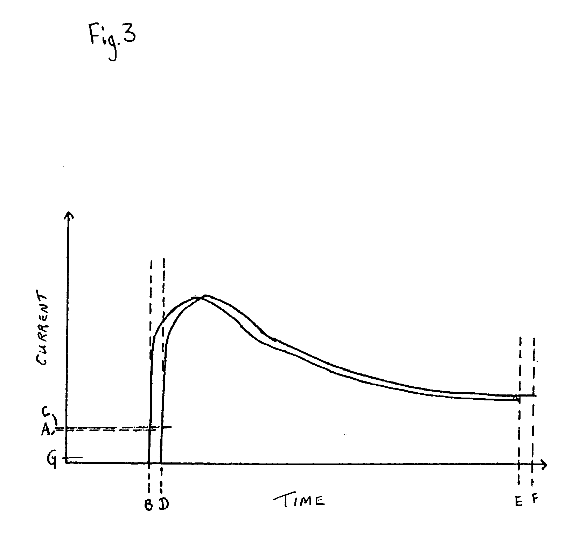

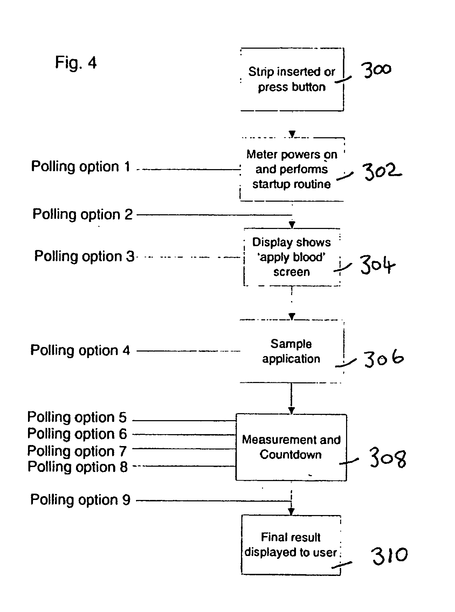

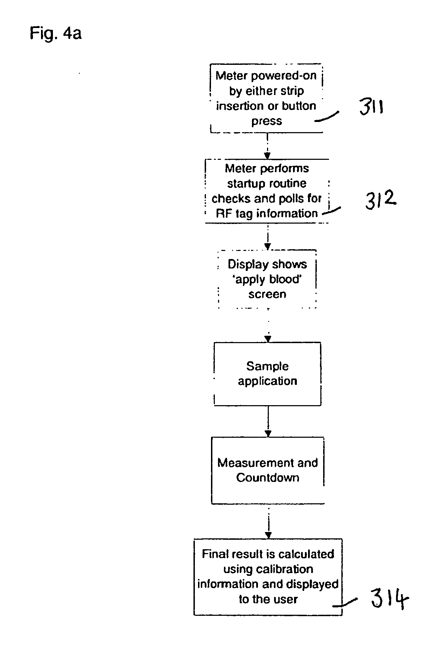

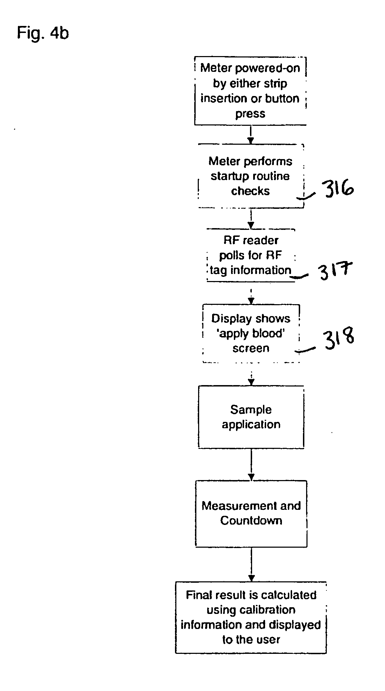

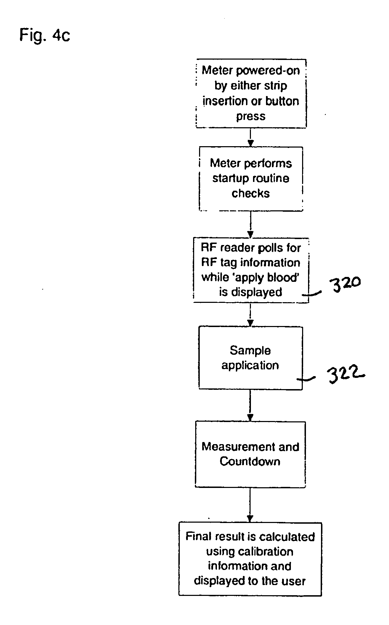

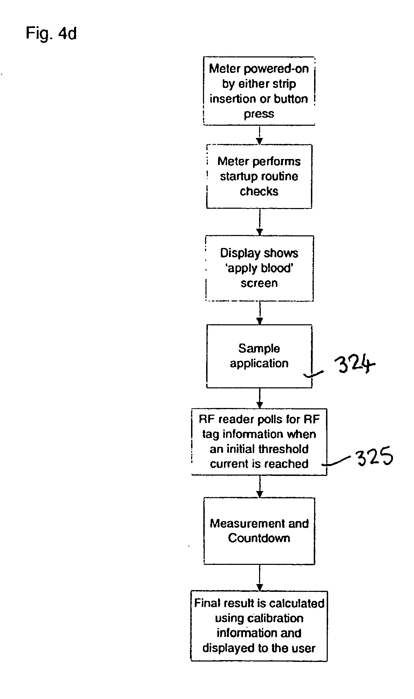

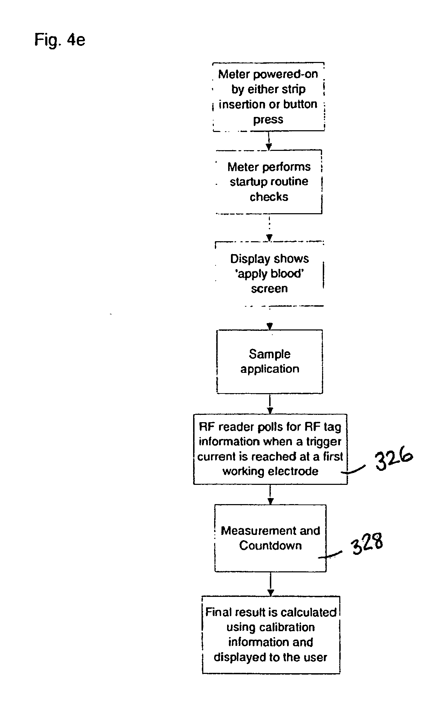

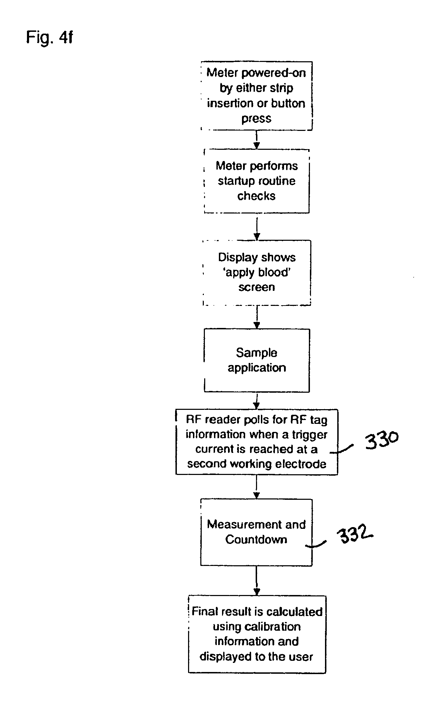

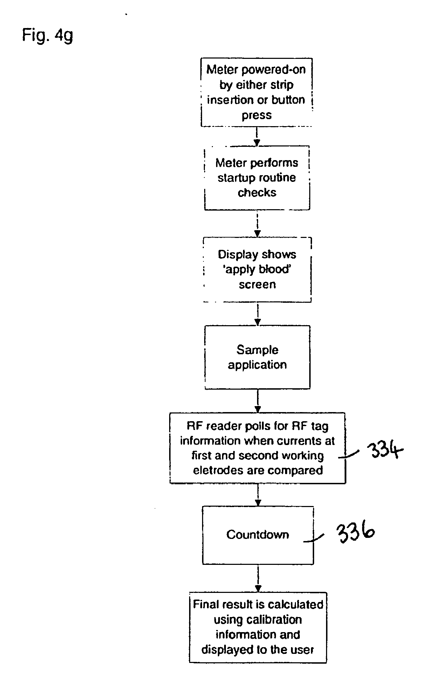

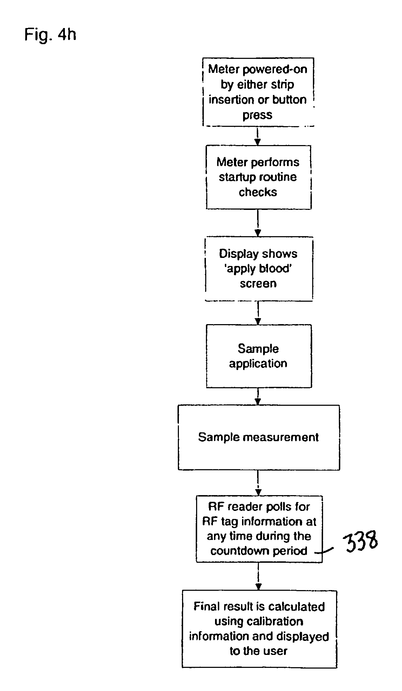

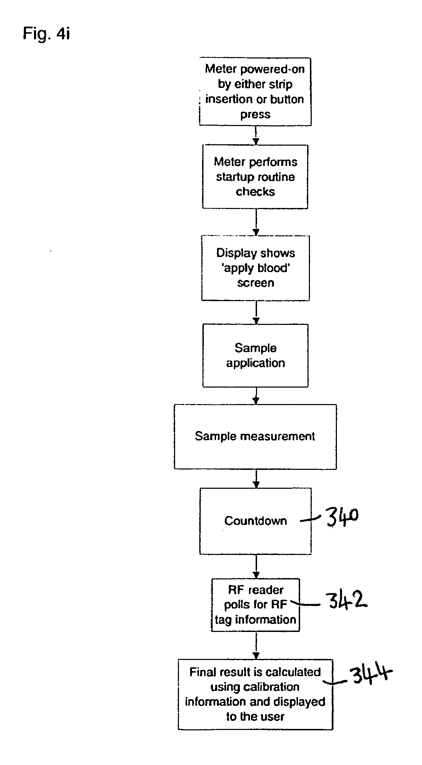

To perform a blood glucose measurement, a user first inserts a new test strip 104 into the strip port connector region 108 of the meter 101. Insertion of a test strip 104, or pressing of a button 105 will initiate the meter to switch on. Software within meter 101 may run a startup routine to check various system components prior to displaying to the user that their meter is ready to begin a test. Vial 102 incorporates an RFID tag 150 that may include information such as the calibration code for the specific batch of test sensors being used. A corresponding RFID reader 152 with appropriate antenna for interrogating RFID tag 150 is located at a suitable position within the housing of meter 101. Polling by RFID reader 152 to determine the presence of RFID tag 150 (and so upload any information) can occur wirelessly at any point prior to the beginning of the measurement calculation in which it is to be used, as will be discussed in more detail in relation to Figures 3 and 4. For example, information stored on RFID tag 150 (such as calibration information) located on vial 102 is required in the calculation performed by the meter software to convert the measured current into an accurate blood glucose concentration and displayed to the user. Wireless transfer of information using RFID may be totally, partially, or not automated. In other words, the transfer can be accomplished without any user interaction other than bringing the vial 102 in close enough proximity to the RFID reader 152 to allow the polling and transfer to take place. Alternatively, the system can be designed to require the user to begin or subsequently acknowledge the polling step to begin and/or complete the information transfer.

RFID provides wireless communication through use of low cost portable data carriers, the technology of which is commonly known and will not be described further herein. An example RFID tag that may be used with the present invention is Tag-it HF-I Transponder inlay (part number RI-103-112A) available from Texas Instruments, [city], Texas, USA. An example RFID reader for use with such an RFID tag is component number TRF 7961 also available from Texas Instruments. Transmission ranges may be in the order or 1 to 30cm depending on power supply and component configuration within the meter, but preferably in the range 1 to 4cm.

Whilst this application refers to RFID tags and RFID readers throughout, other wireless information transfer mechanisms incorporating wireless emitters and receivers could be used such as Bluetooth or WiFi. One advantage of using RFID tags and readers is that the tag can be powered and queried by the reader and does not therefore need its own power source.

Optionally, the exterior housing of meter 101 of system 100 may be designed in such a way to internally integrate a vial or container 102 of test sensors that itself incorporates an RFID tag 150. One such meter is described in patent US5989917 titled "Improved Glucose Monitor And Test Strip Containers For Use In Same" filed February 13, 1996 by Selfcare, Inc. (Attorney Docket number DDI0001), the entire contents of which are hereby incorporated. Such a housing design would hold the RFID reader 152 and tag 150 within the required range for efficient wireless communication. The user may or may not have to physically remove vial 102 from the meter housing to obtain a test sensor to enable them to perform a test. Optionally the user may be able to access and open the vial to retrieve a test sensor whilst the vial is held within the meter housing. An advantage of such a system would be the reduced number of separate components comprising system 100.

Optionally, system 100 may be held within a system kit case, designed specifically to ensure that vial 102 is located in the required position when the reader 152 polls the RFID tag 150 for information such as the calibration code. Such a design may include a holder (for example in-built elastic material or a recess or indent) designed specifically to hold the meter, and also a separate holder for the vial. Such a case design would facilitate the relative positions of the meter and vial, maintaining them in close proximity to each other, and enabling wireless communication there-between.

**Figure 3** shows a plot of a typical analyte measurement timing cycle 200, such as that obtained for the OneTouch® Ultra test strip (available from Lifescan Inc., Milpitas, California, USA).

To perform a blood glucose measurement, the user first inserts a new test strip 104 into the strip port region of their meter 108. The meter software will scroll through a startup sequence, and upon successful completion will display a 'apply blood' icon or message. The user will then lance their skin to obtain a sample of blood to be analysed. The sample is then applied to the test strip, and upon sufficient uptake of sample, the countdown will begin. During the countdown period (5 seconds for the OneTouch® Ultra test strip) an assay procedure is performed by applying +400mV to each of two working electrodes on the test sensor and measuring the current developed after 5 seconds. Almost immediately thereafter, the measured current is transformed, using the calibration information, into a corrected blood glucose concentration.

Turning now to **Figure 3** in detail, timing cycle 200 includes a threshold point G (e.g. a current of 100nA), a trigger point A (e.g. with a trigger current of 150 nA) for a working electrode 1(W1) which triggers the start of a countdown period B (5 seconds in this example), a trigger point C (e.g. with a trigger current of 150 nA) for a working electrode 2 (W2) which triggers the start of a countdown period D (also 5 seconds) for W2, the relative ends of the countdown periods for W1 and W2 are denoted E and F respectively. The current developed at times E (for W1) and F (for W2) are representative of the glucose concentration in the sample under test and are used in a subsequent calculation to determine the glucose concentration in the sample.

Calibration information (for example a calibration code) has to be available to the meter software prior to the beginning of the calculation, to enable the blood glucose concentration to be calculated. If this calibration information is stored on the RFID tag on the vial, then there exist a number of different points throughout the measurement cycle at which transfer of this information could occur so as not to delay the calculation. These points are discussed in relation to **Figure 4.**

**Figure 4** is a simplified process flow diagram, showing the sequence of main events that occur during the process of making an analyte measurement. Several different timing options are indicated for the transfer of information, such as calibration information e.g. a calibration code in particular, from the RFID tag integrated within the vial to the reader in the meter, enabling the software to use this information during the calculation of the final blood glucose result.

Meters such as the OneTouch® Ultra from Lifescan Inc., Milpitas, California, USA, power-on, step 302, automatically when a test sensor 102 is inserted in preparation to perform a measurement. Following strip insertion, step 300, the meter software undergoes a series of start-up checks, step 302, to ensure the meter is ready for use. On completion of the start-up routine, the meter may display a message or icon to indicate to the user that it is ready to accept a sample to be measured, step 304. When a sample is successfully applied, step 306, the assay sequence is triggered, step 308, and the biochemical reaction is measured over a predefined period e.g. 5 seconds. The final measurement is then used in conjunction with the calibration information to calculate the analyte concentration, which is then displayed to the patient, step 310.

Retrieval of the calibration information from an RFID tag, integrated within a vial or container of test sensors as described herein, by RFID technology may occur at several stages during the procedure, outlined by polling options 1 to 9 shown in Figure 4. This application covers all the options outlined although some options are more user friendly and/or power efficient than others. If the RFID tag is not found, and hence the calibration information is not available for whatever reason, then the meter may optionally indicate to the user that the calculation will be performed using the information previously stored, or optionally the user may be provided with the ability to manually calibrate the meter.

**Figure 4a** shows a brief outline of the steps involved in timing of polling option 1. Calibration information may be obtained during the start-up routine. Power-on of the meter in order to conduct a test can occur by means of inserting a test strip into the strip port region or by pressing a button on the user interface, step 311. Before the meter is ready for the user to begin a test, it may automatically perform a start-up sequence of system checks, step 312. Such checks may include memory, pixel illumination, battery charge and ambient temperature for example. The RFID reader within the meter may optionally be programmed to poll for the RFID tag as part of this start-up sequence, thereby ensuring the calibration information and any other relevant information is stored within the memory of the meter and available for calculation of the final result, step 314. This option results in refreshing of the calibration information every time the meter is switched on. This may result in unnecessary power drain on the battery because of the need to power up the reader, as it is not necessary to update the calibration information each time the user performs a test.

**Figure 4b** shows a brief outline of the steps involved in timing of polling option 2. Calibration information may be obtained, step 317, on successful completion of the start-up routine 316 and before the 'apply blood' icon 318 is displayed. The meter software may optionally be programmed to have the RFID reader poll for the RFID tag information automatically after completion of all start-up checks, step 316. If the RFID reader finds the RFID tag, then transfer of the information held in the RFID tag would take only a fraction of a second, thus calibration of the system may be completely invisible to the user. Optionally a confirmation screen, showing the previously retrieved information, may be displayed requiring the user to press a button to confirm that the new information is to be sought. This option could provide some advantage over polling option 1 since polling would not occur if the meter start up checks were unsuccessful.

Whilst it is conceivable for polling options 1 and 2 to include a prompt asking the user to confirm whether they wish to have the meter poll for calibration information each time the meter is switched on, this represents an additional user step, and may result in the user being asked this question needlessly whilst the test sensors in each vial are used up.

Figure 4c shows a brief outline of the steps involved in timing of polling option 3. Calibration information may be obtained whilst the 'apply blood' indicator is being displayed, step 320. Optionally the RFID reader may be programmed to poll for the RFID tag information once, or intermittently during the period in which the system is primed and waiting for sample application to the test sensor, step 322. Polling for the information at this point in the measurement sequence would not increase the overall test time, as essentially the software would be conducting two processes at the same time. Indeed, prior to the 'apply blood' screen appearing 320, a check of the test sensor is made to ensure it is usable (i.e. within certain parameters). If the strip is not usable, then no polling would occur until a usable strip is provided. This would reduce power loss during polling and aid predictability of the lifetime of the battery, as the number of polls would equate to the number of test sensors inserted and found ready to be used. Transfer of calibration from the RFID tag to the meter memory may again be completely invisible to the user, or optionally a confirmation screen may be displayed requiring the user to confirm that new information is to be sought.

**Figure 4d** shows a brief outline of the steps involved in timing of polling option 4. Calibration information may be obtained at a point during sample application, step 324. Optionally, the RFID reader may be programmed to poll for the RFID tag information whilst the user is in the process of applying sample to the reaction zone of the test sensor. Polling may optionally be triggered when a threshold current e.g. 100nA is achieved, step 325, at the start of the electrochemical reaction (indicated by 'G' on Figure 3). Again, this method of calibration would not increase the overall test time, and it may be completely invisible to the user, or optionally a confirmation screen could be displayed.

**Figure 4e** shows a brief outline of the steps involved in timing of polling option 5. Calibration information may be obtained when a trigger current is detected at working electrode 1, step 326. The measurement and countdown period, step 328 (for example a 5 second countdown) is initiated when a particular current e.g. 150nA, is detected at working electrode 1, indicated by 'A' in Figure 3. This trigger current may also optionally be used to trigger the RFID reader to poll for the RFID tag and hence the calibration information contained therein. This has the advantage that the pre-measurement test sensor checks would be complete, and at least the first working electrode (W1) had received sample and was undertaking the assay reaction before polling occurred, therefore polling only occurs when a test sensor is in the meter and is operating correctly. Also, similar to the options already described, obtaining the calibration information at this stage in the measurement cycle would not increase the overall measurement time, and could provide invisible calibration of the system, although optionally a confirmation screen could be displayed.

**Figure 4f** shows a brief outline of the steps involved in timing of polling option 6. Calibration information may be obtained when a trigger current is detected at working electrode 2, step 330. Similar to timing of polling option 5, a trigger current measured at working electrode 2 e.g. 150nA (indicated by 'C' in Figure 3) could be used to trigger the RFID reader located within the meter to poll for the RFID tag. As working electrode 2 is located further down the test sensor than working electrode 1 i.e. further away from the inlet for sample application, then the trigger current for W2 (indicated by 'D' in Figure 3) is reached approximately 300ms after the trigger current for W1 (indicated by 'B' in Figure 3), and the countdown period, step 332 for W2 is initiated. This is also an indicator that the test sensor has been constructed correctly and it is likely that an appropriate amount of blood has been used so that a successful fill of the test sensor is likely to occur because sample has reached the second working electrode. Therefore in this option, no polling (and hence no battery usage) would occur if the sample does not reach the second working electrode.

**Figure 4g** shows a brief outline of the steps involved in timing of polling option 7. Calibration information may be obtained at any time during the countdown. Here the option is to program the RFID reader to poll for the RFID tag information at any point within the countdown period. The countdown period for the OneTouch® Ultra meter (available from Lifescan Inc., Milpitas, USA) is 5 seconds, allowing sufficient time for the RFID reader to poll for the RFID tag and obtain the calibration information.

**Figure 4h** shows a brief outline of the steps involved in timing of polling option 8. Calibration information may be obtained once the process carried out to check for sufficient fill of the test sensor is complete, step 338. As described in polling option 6, the sample e.g. blood takes some time to move up the reaction zone of the test sensor by capillary action. At the end of the countdown period, step 336, the measurement software may determine that the strip is operating correctly, has no manufacturing errors and has received sufficient sample to perform the analysis, by comparing the currents detected at W1 and W2 (times E and F respectively in Figure 3) and checking that the two measurements are within acceptable agreement with one another e.g. +/-20%. If the test strip is not performing correctly e.g. if there is a manufacturing error or insufficient sample was applied, then this calculation may prompt an error message to warn the user and may ask them to retest. Once it has been determined that the test sensor is performing correctly, only then is the RFID reader activated to poll for the RFID tag. The RFID reader may optionally be programmed to poll for the RFID tag information during, or just after this sensor performance calculation. Again, the process of calibration would not increase the overall measurement time, and may be completely invisible to the user. If polling occurs after the sensor performance calculation, there is reduced risk of polling unnecessarily. Alternatively, by having already obtained the necessary information by polling for the RFID tag in advance of inviting the user to apply sample to the test sensor (as described in polling options 1 and 2), there is reduced risk of wasting a test sensor should the RF polling for the calibration information, and the optional manual entry of the calibration information both be unsuccessful for any reason.

**Figure 4i** shows a brief outline of the steps involved in timing of polling option 9. Calibration information may be obtained, step 342, after the end of the current measurement, step 340, but before the beginning of the calculation that transforms the measured current into an accurate blood glucose result, step 344. The RFID reader may optionally be programmed to poll for the RFID tag to obtain the calibration information after the check for sufficient sample has occurred and the current has been measured. The calibration information is required in the calculation of the final result subsequently displayed to the user therefore it is retrieved from the RFID tag prior to the start of this calculation. This option can take longer since there is no advantage from conducting activities e.g. countdown and polling in parallel.

Provided the RFID reader is instructed to poll the RFID tag for the calibration information during one of the above-listed options, then the calibration information will be readily available to be used in the calculation of the analyte concentration.

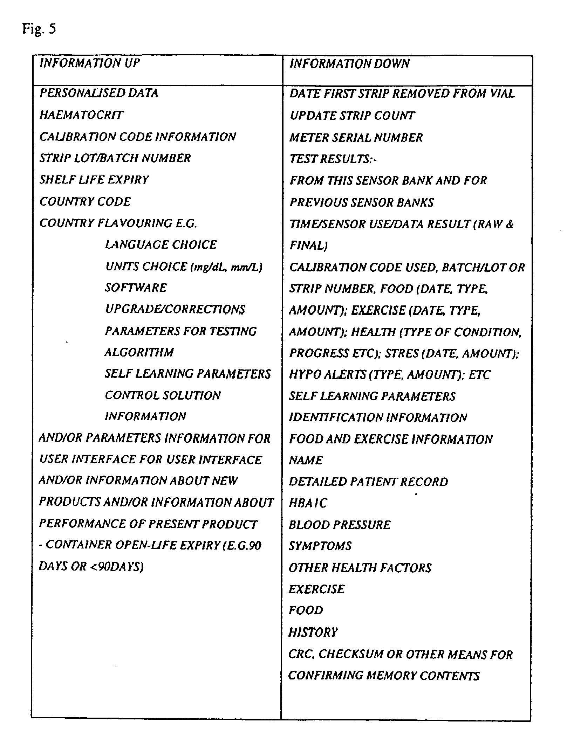

**Figure 5** shows a table of information that may be loaded from a RFID tag to the meter and/or from the meter to the RFID tag, in accordance with any of the example embodiments of the present invention.

Four different example embodiments incorporating the present invention will now be discussed.

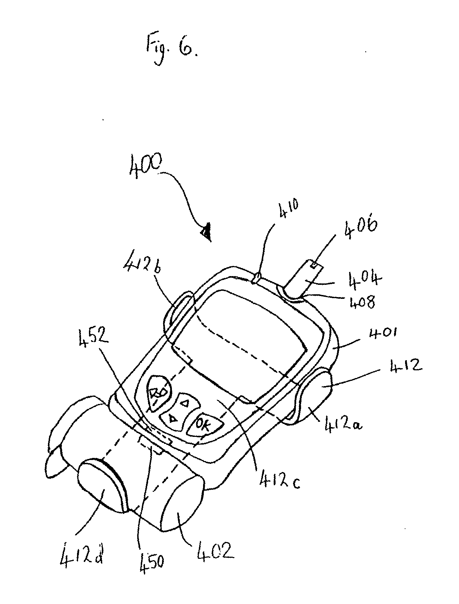

**Figure 6** shows a first example embodiment of a system 400 according to the present invention, including a meter 401 incorporating an RFID reader 452, a vial 402 incorporating an RFID tag 450, a test sensor 404, a reaction zone 406, a strip insertion port 408, an indicator 410 and a clip 412 comprising an elongate portion 412c and gripping features 412a, 412b and 412d. Clip 412 is semi-rigid and designed to releasably receive meter 401 (between gripping features 412a and 412b) and vial 402 (via gripping feature 412d). Thus both the meter 401 and vial 402 can be held in fixed relation together by clip 412. Whilst in clip 412, both the front face and strip insertion point 408 of meter 401 can be accessed by a user. Also, whilst in clip 412, vial 402 can be accessed by a user. Typically gripping features 412a, 412b and 412d provide an interference snap-fit with meter 401 and vial 402 and are releasable.

**Figure 6 i**s an example embodiment of an analyte-monitoring device, such as a glucose-monitoring device for example, used by diabetic patients to measure their blood glucose concentration. System 400 comprises a meter 401 and a vial of test sensors 402. According to the present invention, meter 401 includes an RFID reader 452 typically comprising an antenna, transceiver and decoder, located within the meter housing. Vial 402 includes a transponder, commonly known as an RFID tag 450, electronically programmed with information such as calibration data, and optionally expiry and other country-specific information such as the examples provided in Figure 5. Methods of integrating the RFID tag 450 as part of a vial of test sensors is described in detail in co-pending application 'Container with RFID device for storing test sensors' (DDI5116GBPSP, filed at the UK Patent Office on 22 December 2005 in the name of LifeScan Scotland Ltd).

System 400 includes a locator, here in the form of a clip 412 to hold vial 402 fixed in relation to meter 401 thereby providing the close proximity required for efficient RFID communication. In one embodiment, clip 412 is molded in one piece using a semi-rigid, yet slightly deformable material. Clip 412 is essentially 'T'-shaped, and is intended to grip meter 401 from behind by means of two optionally rounded gripping elements 412a and 412b, that press against the top surface of meter 401 at each side, securely holding meter 401 against elongate portion 412c of clip 412. In this embodiment, gripping elements 412a and 412b may optionally engage with cooperating features located on the meter housing 401 (not shown). Elongate portion 412c terminates into a further gripping element 412d, that extends beyond the bottom edge of meter 401. Gripping element 412d is intended to hold vial 402 securely adjacent the bottom edge of meter 401, enabling successful RF communication between the RFID tag 450 integrated within vial 402 and the reader 452 housed within meter 401. It will be apparent to a person skilled in the art that other embodiments (i.e. materials, shape and components) may be used to provide a locator to hold a vial and meter in close proximity to one-another, and these are intended to be included. Alternatively, a meter housing may be adapted e.g. by provision of a recess, to receive a vial of test sensors, see US5989917 titled 'Improved glucose monitor and test strip containers for use in same' filed February 13, 1996 in the name of Selfcare Inc., (Attorney Docket number DDI 0001), the entire contents of which are hereby incorporated

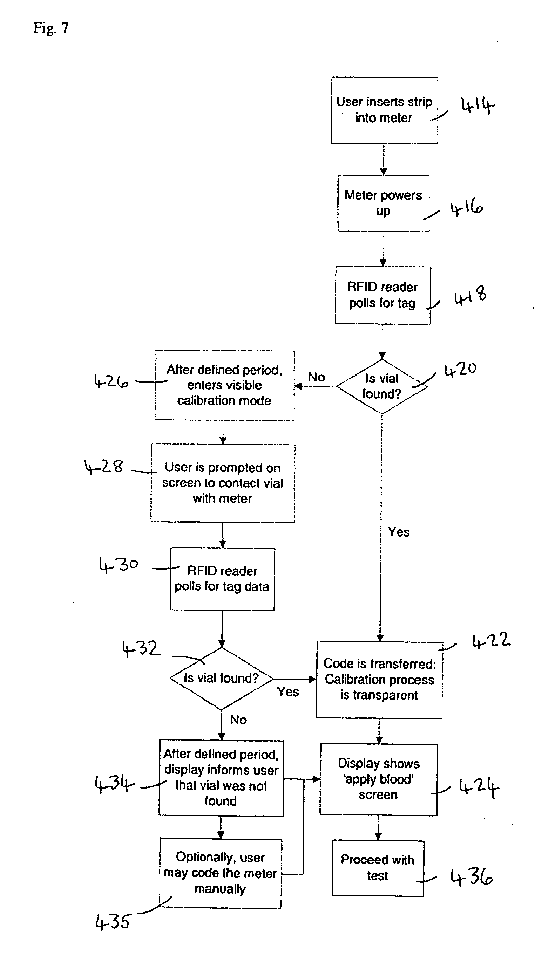

The following description utilizes the embodiment shown in Figure 6 in combination with timing of polling option 2 described in relation to Figure 4 and 4b, and will be described in more detail with respect to Figure 7. To perform a test, a user would remove a test sensor 404 from within vial 402, optionally whilst vial 402 is held within clip 412, or alternatively prior to attaching vial 402 to clip 412, then insert strip 404 into insertion port 408. The act of inserting strip 404 into port 408 may power-on meter 401, automatically initiating the test procedure. Optionally a splash screen may first be displayed, followed by a display check. Following the power up procedure, the RFID reader 452 housed within meter 401 emits a radio frequency signal to activate RFID tag 450 located on vial 402. The reader controls data acquisition and communication, and decodes the information stored within the integrated circuit of RFID tag 450 incorporated within vial 402.

Clip 412 of the present invention therefore ensures that vial 402, with RFID tag 450 housed therein, is positioned correctly to receive the RF signal sent by antenna 452, enabling transfer of information such as the calibration code to the meter software prior to the user applying blood to the reaction zone 406 of test sensor 404. System 400 of the present invention provides a unique, low power consumption hence power efficient and cost-effective means of invisible calibration to the user.

Optionally, clip 412 may be used alone or in conjunction with a specifically designed system kit case. The process steps of performing a test such as a blood glucose measurement will be described in more detail in relation to Figure 7.

**Figure 7** shows a flow diagram of the process steps involved in calibrating system 400 of Figure 6. To perform a measurement, the user first inserts a test sensor into insertion port, step 414, which may optionally power-on meter 401 automatically, step 416. At a defined point during the power-up sequence, the RFID reader 452 located within the housing of the meter is programmed to start emitting wirelessly, at a predetermined frequency to poll for RFID tag 450, step 418. At this stage in the sequence, the meter software has to determine whether a RFID tag 450 has been located or not, step 420. If a vial containing an RFID tag is within range and located within the short polling period, then the calibration code and optionally any other relevant pieces of information is transferred from the tag to the meter software, step 422, a process invisible to the user. As soon as a valid tag is detected and the information obtained, the RFID circuitry is switched off to conserve battery power. Following successful calibration, meter 401 will move to the 'apply blood' instruction screen, step 424, and the user will then be able to proceed with the measurement procedure, step 436, assured that their meter is correctly calibrated for the vial 402 of test sensors they are using. Optionally a confirmation screen displaying the calibration may be displayed briefly to the user, and optionally the user may be asked to confirm the calibration code.

If however an RFID tag 450 is not located at step 420 by the reader during the short polling period 418, then meter 401 may enter a visible calibration mode, step 426. A message or indicator may be displayed to the user, step 428, for a short period of time (e.g. 2 to 10 seconds) informing them that the tag was not found. During this period, RFID reader 452 may optionally continue to poll for RFID tag 450, step 430, and if vial 402 is placed into clip 412 then RFID tag 450 will be located and the calibration information transferred, step 422. If however the RFID tag 450 is still not found, then a message may be displayed on the screen that no vial was found, step 434. The RFID circuitry only polls for the RFID tag information briefly. Thereafter the RFID circuitry times out and switches off to conserve battery power, and meter 401 retains the last calibration code that was used. The 'apply blood' message or indicator is then displayed to the user, step 424, allowing the patient to carry on with the test, step 436, however this is with the knowledge that the calibration code may not be correct. Optionally, the user may be provided with the facility to manually enter the correct calibration code, step 435, if for some reason the RFID information transfer is unsuccessful. This will allow the user to continue testing, and ensure that the result obtained is accurate.

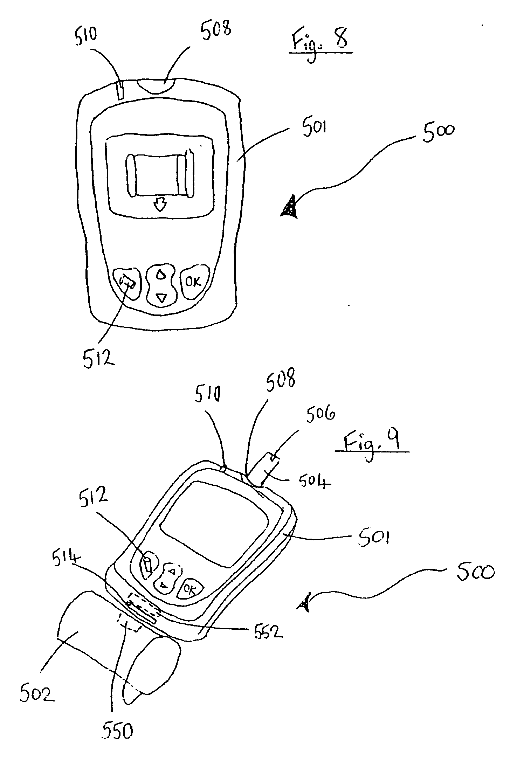

**Figure 8** shows an example analyte measuring system 500 according to a further embodiment of the present invention, including a meter 501 incorporating an RFID reader (not shown), a strip insertion port 508, a first optional indicator 510 and a dedicated calibration button 512.

**Figure 9** shows a perspective view of the system 500 of Figure 8, showing a meter 501 incorporating an RFID reader 552, a vial 502 incorporating an RFID tag 550, a test sensor 504, a reaction zone 506, a strip insertion port 508, a dedicated calibration button 512 and a second optional indicator 514.

Referring now to Figures 8 and 9, provided is a further example embodiment of an analyte measurement system 500 according to the present invention. As described previously in relation to Figure 6, meter 501 contains an RFID reader 552 housed therein, and vial 502 contains an RFID tag 550 either co-molded within the make-up of vial 502 or optionally as part of the label provided on vial 502, as described in detail in co-pending application DDI 5116GBPSP 'Container with RFID device for storing test sensors', filed December 2005.

System 500 enables a user to ensure their meter is correctly calibrated at any time i.e. the procedure of calibration does not require the user to insert a new strip into the receiving port to power up the meter. The same calibration procedure will apply whether the meter 501 is powered on by depressing a button or by inserting a strip 504. In this example embodiment, calibration of a new vial 502 of test sensors is enabled by means of the user bringing vial 502 close to meter 501 and pressing a dedicated button 512. Button 512 may have the exclusive purpose of activating the RFID reader and thus polling for RFID tag 550. Button 512 may be pressed either when meter 501 is in an off mode, or after a sensor 504 has been inserted into port 508 or meter 501 is otherwise switched on. However, typically button 512 must be pressed prior to application of sample to sensor 504 to allow polling for an RFID tag and subsequent uploading of information such as calibration information. It is possible for button 512 (and hence polling) to be activated during the 5 second countdown, but this would require a prompt for a user action within a defined time window and therefore this could delay the test result if it were not done in a timely manner.

An additional indicator 514, such as an LED may also be used to indicate the location of the area of meter 501 containing the RFID reader so providing guidance to the user as to where to place vial 502 to be in close proximity to RFID reader 552. Indicator 514 may also provide information on the communication status by either illuminating, ceasing to illuminate or flashing at a certain time for example.

From the sleep or off mode, meter 501 is activated either by pressing button 512 or inserting a test strip 504 into receiving port 508 prior to pressing button 512. Following an optional splash screen and display check, the calibration code previously stored in meter 501 may be displayed, followed by a display indicator showing that reader 552 is polling for RFID tag 550. The display indicator may optionally be combined with a second indicator such as illumination of an LED 510 for example, to indicate that the antenna of RFID reader 552 within meter 501 is emitting a radio signal to scan for the presence of RFID tag 550.

Dedicated button 512 may include an icon, such as a picture of a vial in this instance, to clearly and unambiguously depict its use. A user would intuitively know that dedicated button 512 is involved in the procedure for calibrating system 500, particularly when coupled with similar icons displayed on the user interface of meter 501.

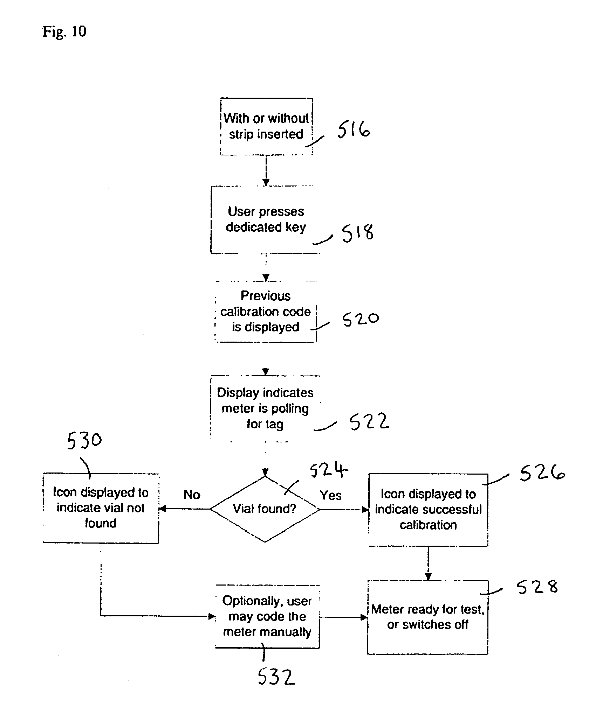

Figure 10 shows a flow diagram of possible steps involved in calibrating system 500 of Figures 8 and 9. A user is able to calibrate their system 500 with or without actually performing a measurement, step 516. With a strip inserted into the insertion port or not, the user presses the dedicated key or button at any time as long as it is prior to blood application, step 518, similar to polling timing options 1 and 2 described in relation to Figure 4. The previously stored calibration code may then optionally be displayed to the user, step 520. The display will then indicate that reader 552 housed within meter 501 is scanning for the RFID tag 550 located within vial 502, step 522. Such a display may be by means of a pictorial representation, an intuitive icon, words or even LED state. If the vial is detected, step 524, an icon may be displayed to the user to indicate successful location of RFID tag 550 and/or successful transfer of information e.g. calibration information, step 526, and the meter is then ready to be used to perform a test or power off, step 528. Following successful transmission and receipt of the information stored, the RFID circuitry may switch off automatically to conserve battery power.

If RFID reader 552 does not communicate with an RFID tag 550, then an icon may be displayed to the user to indicate that no vial was found, step 530, and optionally the previous calibration code stored within the meter memory may again be displayed. The meter is then ready to begin a test using the previously stored code, or if not required it will power off, step 528. As described previously, if for some reason the RF auto-calibration is unsuccessful, the user may be provided with the option to manually calibrate their system, step 532, to ensure accuracy of readings taken.

RFID reader 552 will only activate for short periods of time in order to conserve battery power. A specific time-out will be programmed to ensure the RF circuitry powers off after the predetermined period of time. Optionally, button 512 may be operated by means of a depress and hold action, thereby the possibility of accidentally powering on meter 501 and activating the RF circuitry unnecessarily is virtually eliminated, in-keeping with the desire to conserve battery power.

Provision of a dedicated button 512 for calibrating system 500 according to the present invention, enables a user to more easily maintain the correct calibration status of their meter. A dedicated button 512 may also be used in conjunction with clip 412 seen in Figure 6.

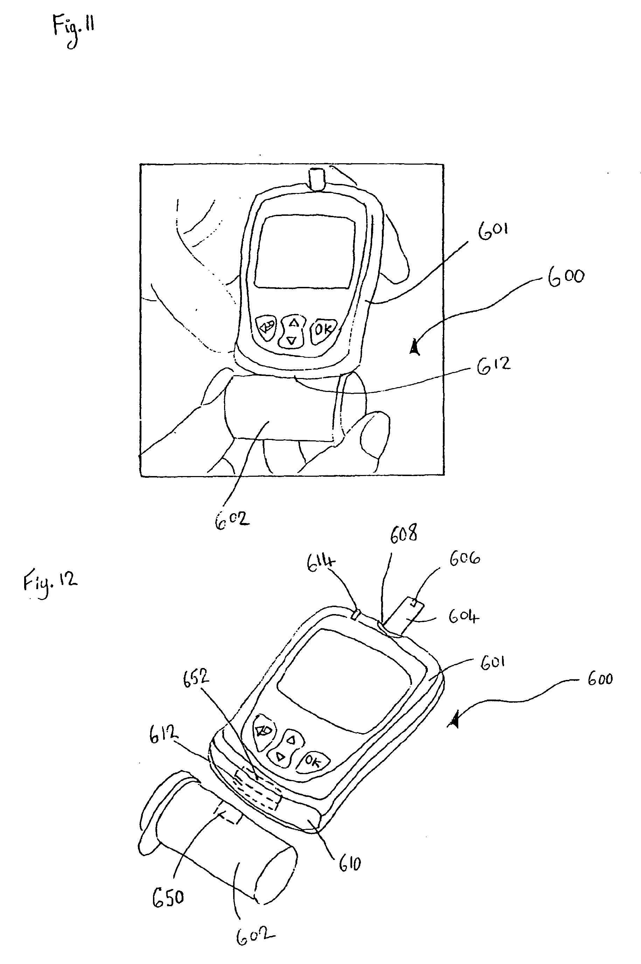

**Figure 11** shows an example system 600 according to a further embodiment of the present invention, including a meter 601, a vial 602 and a micro-switch 612.

**Figure 12** shows a perspective view of the system 600 of Figure 11, including a meter 601 incorporating an RFID reader 652, a vial 602 incorporating an RFID tag 650, a test sensor 604 with a reaction zone 606, a strip insertion port 608, a concave recess 610, a micro-switch 612 and an indicator 614. An example micro-switch is an ultra miniature button micro-switch, component number DH3C-B1AA available from Cherry Electrical Products Ltd., Luton, England. In one embodiment of micro-switch 612, a magnet (not shown) and RFID tag 650 are incorporated within vial 602, and a cooperating reed switch (not shown) is incorporated within meter 601. When vial 602 is placed in close proximity to meter 601, the magnet triggers the reed switch thereby activating RFID reader 652 to poll for RFID tag 650 and transfer the information.

In more detail, an RFID tag 650 and a magnetic element (not shown) is associated with a vial 602 of test sensors, either via application as a label or integrated within the molding of vial 602. The magnetic element should be in close proximity to RFID tag 650. A reed switch is incorporated within meter 601 in a suitable location e.g. on the top surface, side or base of meter 601. To power up and calibrate system 600, the user would positively place and hold vial 602 against a conveniently marked target area on meter 601 where the reed switch is located. The proximity would be such that the magnet within vial 602 triggers the reed switch to turn on meter 601 and activate the RFID circuitry. RFID reader 652 would poll for a short period of time e.g. 2 seconds, interrogate RFID tag 650 and subsequently transfer the calibration code and any other information to meter 601. Once the information is retrieved the RFID circuitry is immediately switched off, and some form of feedback may be given to the user indicating that they may stop holding vial 602 against meter 601. The calibration information retrieved may be displayed to the user for verification, before the user is given the option of whether or not they wish to proceed with a test.

Meter 601 may optionally be switched on to the data management mode by pressing the on/off button, and doing so will not power up the RFID circuitry. Optionally, insertion of a test sensor into meter 601 may power on meter 601 without activating the RFID circuitry, whereby the calibration information may still be entered manually. If meter 601 was switched on via micro-switch 612 and no response from an RFID tag 650 was detected after polling for a short, set period of time e.g. 2 seconds, the RFID circuitry would power off and manual input of the calibration information may be requested or meter 601 may switch off entirely. This may occur if there was an error with RFID tag 650, an inconsistent placement of vial 602 to meter 601 or a rogue magnetic source potentially triggering micro-switch 612 without a vial 602 present.

Referring now to Figures 11 and 12, provided is a further example embodiment of an analyte measurement system 600 according to the present invention. As described previously in relation to Figures 6, 8 and 9 meter 601 contains an RFID reader 652 housed therein, and vial 602 contains an RFID tag 650 either co-molded within the make-up of vial 602 or optionally as part of the label provided on the vial, as described in detail in co-pending patent application 'Container with RFID device for storing test sensors' (DDI5116GBPSP, filed at the UK Patent Office on 22 December 2005 in the name of LifeScan Scotland Ltd). Similar to analyte measurement system 500 described in relation to Figures 8 and 9, system 600 also enables a user to ensure their meter 601 is correctly calibrated at any time i.e. with or without a test strip 604 inserted into insertion port 608.

In this example embodiment, the user touches vial 602 against a specific location on the external housing of meter 601, e.g. a specially designed locator arrangement such as concave recess 610, to enable calibration of a new vial 602 of test sensors. Concave recess 610 may be shaped in the negative form of vial 602, making it intuitive to the user to hold vial 602 against this area of meter 601. Optionally, an additional indicator may be provided to the user, for example by means of colouring and/or illuminating active area 610 of meter 601 in a distinctive way, or the addition of a label including text or a picture to encouraging the user to bring the vial into contact with meter 601 at this specific location. Optionally, active area 610 may include a second LED 612 to provide further information regarding the status of the communication.

Contacting the meter at concave recess 610 activates a micro-switch 612 that in turn initiates the RFID reader 652 to poll in search of RFID tag 650. Micro-switch 612 may have the exclusive purpose of activating the RFID circuitry and thus calibrating the meter.

An indicator 614, such as an LED indicator for example, provided on the external housing of meter 601 may be used to give additional information regarding the status of the RF communication. Such an indicator may illuminate or flash to confirm that the antenna within meter 601 is emitting a radio signal to scan for the presence of an RFID tag. Similarly, extinction of such an indicator, or an obvious change in some other way, may supplement the screen display indicating to the user that calibration has been successful. It would be apparent to a person skilled in the art that different indicators may be used to reflect the status of the wireless communication, and is not intended to be restricted to those described herein.

From the sleep or off mode, meter 601 is activated either by contacting vial 602 against micro-switch 612 located within concave recess 610 on the external housing of meter 601, or by inserting a test strip 604 into receiving port 608. Following an optional splash screen and display check, the calibration code previously stored in meter 601 may optionally be displayed, followed by a display indicator showing that the meter is scanning for the RFID tag 650 to obtain the calibration information. RFID readers 452, 552 and 652 and RFID tags 450, 550 and 650 depicted herein are shown by means of example only, and are not intended to restrict the size or location of either component covered within the realms of this disclosure.

Optionally, system 600 according to the present invention may be used as described or in conjunction with a specifically designed case.

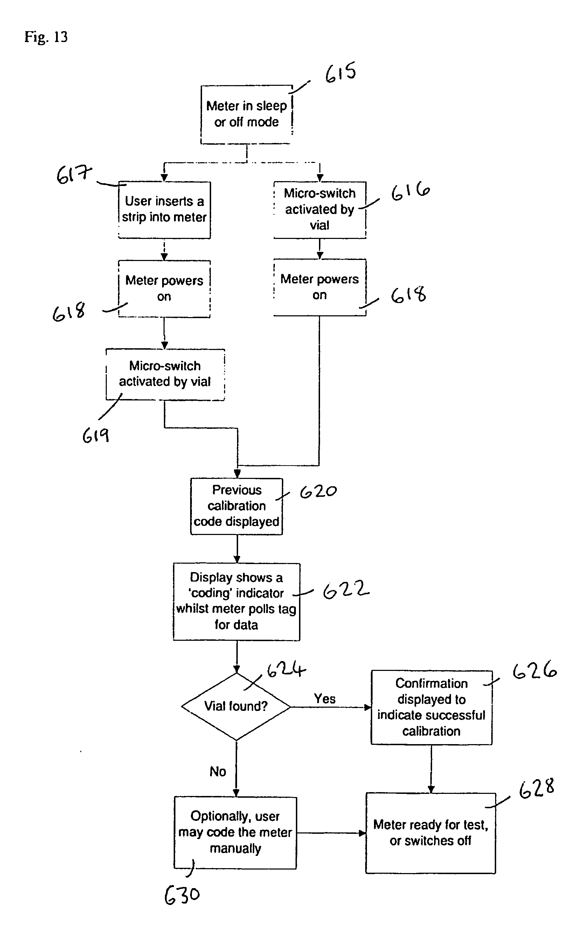

**Figure 13** shows a flow diagram of the process steps involved in calibrating the system 600 of Figures 11 and 12. When meter 601 is in off or sleep mode 615, the user may insert a test sensor, step 617, if they intend to make a measurement. Inserting the strip may automatically power-on the meter, step 618, and the meter would display a prompt requesting the user to contact micro-switch 612 with vial 602, step 619. Alternatively system 600 can be calibrated without a test sensor being inserted, by contacting micro-switch 612 within recess 610 with vial 602, step 616. Activation of micro-switch 612 causes meter 601 to power-on, step 618, and the previous calibration code stored within the meter memory may optionally be displayed, step 620, prior to RFID reader 652 polling for RFID tag 650 to retrieve information such as the calibration information, step 622.

Whether meter 601 is powered-on by strip insertion or activation of micro-switch 612, the meter first displays the last calibration code saved in the meter memory, step 620. After a predefined period of time, an indicator is displayed showing that reader 652 housed within meter 601 is polling for the information stored in the RFID tag 650 located within vial 602, step 622. If a vial is found, step 624, a confirmation is displayed to indicate successful calibration, step 626, and the meter is ready to begin a test or alternatively power-off if not required, step 628. In this embodiment, system 600 incorporates polling timing options 1 or 2 described in relation to Figure 4, retrieving the calibration information from RFID tag 650 prior to sample application. If a vial is not found, or it is removed without successful communication, step 624, then meter 601 may still be used for a test using the calibration code previously stored in the memory. If the user does not intend to test at this time, then meter 601 may return to the off or sleep mode, step 628. The user may be provided with the option to manually calibrate system 600, step 630, enabling them to continue with the test and ensures accurate results.

By utilizing a reed switch and magnet in this embodiment, the user action of presenting tagged vial 602 to meter 601 combines power up of system 600 with calibration. This embodiment therefore has the advantage of being efficient with regard to polling time and hence power consumption.

As described in relation to earlier embodiments, the RF circuitry will only poll for the RFID tag information for a short, defined period of time in order to conserve battery power. The predefined period would be programmed into the meter software, causing the RF circuitry to power-off independent of whether a tag was found or not. Controlling the activation of the RF circuitry for such short periods of time is beneficial in the potential occurrence of micro-switch 612 being triggered accidentally, for example whilst being carried in a bag or pocket. An automatic RF circuitry activation time-out mechanism will maximize battery power conservation.

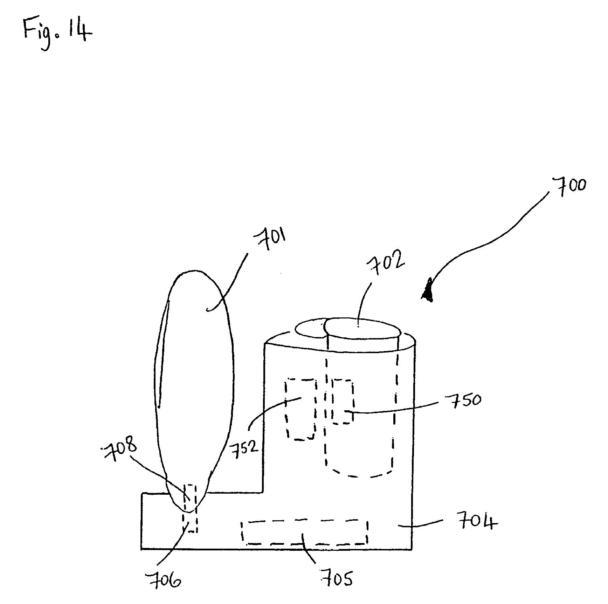

**Figure 14** shows an example system 700 according to a further embodiment according to the present invention, including a meter 701, a stereo jack connector 708, a vial 702 incorporating an RFID tag 750 therein, a cradle 704 including an RFID reader 752, a battery 705 and a cooperating engagement feature 706.

This last example embodiment provides a means for enabling meters currently commercially available to be implemented with RFID auto-calibration technology as described herein. Figure 14 shows a conventionally available meter 701, such as the OneTouch® Ultra meter (available from Lifescan Inc., Milpitas, USA.), held in close proximity to a vial of test sensors 702 that incorporates an RFID tag 750 by means of a cradle 704. Cradle 704 includes an RFID reader 752 located in a position close to the engagement point for vial 702, hence in close proximity to RFID tag 750 to facilitate wireless communication there-between. Meter 701 is attached to cooperating engagement feature 706 of cradle 704 via the stereo jack opening 708 in this example embodiment. Optionally, meter 701 may engage to cradle 704 via the strip port connector, or optionally via any type of connector e.g. USB. Figure 14 provides one example embodiment of a cradle according to the present invention; it would be apparent to a person skilled in the art that different shapes, forms and materials of cradle are conceivable.

It is intended that cradle 704 be used each time a user purchases a new vial 702 of test sensors, enabling easy, quick and reliable calibration of their system 700 prior to use for measuring their blood glucose concentration. Placement of vial 702 into cradle 704 may trigger the RFID reader 752 within cradle 704 to poll for RFID tag 750 and retrieve the information stored therein. Placement of meter 701 into cradle 704 engages electronic communication between cradle 704 at engagement feature 706, with stereo jack connector 708, enabling transfer of information such as calibration information to meter 701. Transfer of the information from vial 702 would occur once for each new vial, when vial 702 and meter 701 were placed in cradle 704. Thus the information would be available before any subsequent measurements and calculations of results using said measurements.

Cradle 704 would contain all necessary electronics required to read the information stored on RFID tag 750, retrieve this information, then interrogates the appropriate parameters within the memory of the meter and modifies them to include the correct information corresponding to the vial of test sensors being used. It is anticipated that cradle 701 may also include a power source 705, and may optionally include an external indicator (not shown) such as an LED for example, that can be used to inform the user of battery charge status, thereby informing them when they need to change the battery. Optionally power source 705 may be rechargeable, therefore an external indicator may display the charging status to the user.

Such a cradle enables use of RFID technology for auto-calibration with existing meters and/or future meters without inbuilt RFID technology e.g. lower cost meters whereby the cradle may be provided as an accessory. Auto-calibration by means of RFID technology provides the user with an easier and more reliable process than the conventional manual process.

Calibration information corresponding to the specific batch of test sensors would be contained within RFID tag 750 and transmitted wirelessly to the memory of meter 701 upon request from the RFID reader 752 housed within meter 701. Other information may also, optionally, be transferred between vial 702 and the memory of meter 701, examples of such information are listed in Figure 5.

The limitations associated with using RFID technology to facilitate the transfer of calibration information, namely the limited read range and the limited available battery power, are in whole or in part overcome by the embodiments provided herein. Each embodiment ensures that the meter and vial, or container housing new test sensors, are within the limited read range of the RFID reader. Each embodiment may also ensure that the RFID circuitry is only powered-on for short periods when required, and automatically powers-off following successful transfer of stored data to conserve battery power. RFID auto-calibration may also provide the user with fewer steps in the process of performing a blood glucose measurement, and may reduce the overall test time if information transfer is completely invisible to the user.

It should be understood that various alternatives to the embodiments of the invention described herein might be employed in practicing the invention. It is intended that the following claims define the scope of the invention and that methods and structures within the scope of these claims and their equivalents be covered thereby.

The following is a non-exhaustive list of embodiments of the invention that are or may be claimed.
1. A method for measuring a characteristic of an analyte or indicator in a body fluid in a system comprising a meter and a separate test sensor container, the method comprising:
   a) measuring at least one quantity representative of the characteristic of the analyte or indicator;
   b) polling for a test sensor container;
   c) on successful polling wirelessly transferring information from the test sensor container to the meter and;
   d) calculating the characteristic of the analyte or indicator using said quantity and the information
   and in which step (c) is complete before the start of step (d).
2. A method according to embodiment 1 in which step (c) is complete before the end of step (a).
3. A method according to embodiment 1 or 2 in which step (c) is complete before the start of step (a).
4. A method according to any preceding embodiment comprising the step of (e) comparing two quantities representative of the characteristic of the analyte or indicator and in which step (b) commences after the result of step (e) is known.
5. A method according to any preceding embodiment in which step (a) includes counting down a number of seconds and step (c) commences during the count down.
6. A method according to embodiment 5 in which step (b) commences during the count down.
7. A method according to any preceding embodiment in which step (c) commences once a trigger level of a first predetermined quantity is reached.
8. A method according to embodiment 7 in which step (b) commences once a trigger level of a first predetermined quantity is reached.
9. A method according to embodiment 7 or 8 in which the first predetermined quantity is a first working electrode current.
10. A method according to embodiment 7, 8 or 9 in which a count down commences once the trigger level is reached.
11. A method according to any preceding embodiment in which step (c) commences once a trigger level of a second predetermined quantity is reached.
12. A method according to embodiment 11 in which step (b) commences once trigger level of a second predetermined quantity is reached.
13. A method according to any preceding embodiment in which second predetermined quantity is a second working electrode current.
14. A method according to embodiment 11, 12 and 13 in which the count down commences once the trigger level is reached.
15. A method according to any preceding embodiment in which step (b) commences once an initial threshold current is reached.
16. A method according to embodiment 13 in which the initial threshold is ≤ about 100nA.
17. A method according to any preceding embodiment comprising a step (f) displaying an "Apply Sample" request and one of step (b) and step (c) commences during step (f).
18. A method according to any preceding embodiment comprising step (f) displaying an "Apply Sample" request and one of step (b), step (c) is complete before step (f) commences.
19. A method according to any preceding embodiment comprising step (g) performing meter start up routine and one of step (b) and step (c) commences during step (g).
20. A method according to any preceding embodiment comprising step (g) performing meter start up routine and one of step (b) and step (c) is completed before the end of step (g).
21. A system comprising a meter and a separate test sensor container adapted to carry out a method according to any of embodiments 1 to 20.
22. A system comprising a meter, a separate test sensor container and a clip, having two receiving portions each for receiving one of the meter and the test sensor container the clip being adapted to hold the meter and test sensor container in fixed relation to one another.
23. A system according to embodiment 22 adapted to carry out a method according to any of embodiments 1 to 20.
24. A system according to embodiment 22 or 23 in which the clip is adapted to releasibly receive one or both of the meter and test sensor container.
25. A system comprising a meter and a separate test sensor container the meter comprising an activation mechanism to commence polling for the test sensor container.
26. A system according to embodiment 22 in which the activation mechanism comprises a button.
27. A system according to embodiment 22 in which the activation mechanism comprises a microswitch.
28. A system according to embodiment 24 in which the test sensor container comprises a magnet and the meter comprises a read switch.
29. A system according to embodiment 22 to 27 further adapted to carry out a method according to any of embodiments 1 to 20.
30. A system or method substantially as described herein with reference to and/or as illustrated in the accompanying figures.

## Claims

1. A system comprising a meter, a separate test sensor container and a clip, having two receiving portions each for receiving one of the meter and the test sensor container the clip being adapted to hold the meter and test sensor container in fixed relation to one another.

2. A system according to claim 1 adapted to carry out the following steps:
a) measuring at least one quantity representative of the characteristic of the analyte or indicator;
b) polling for a test sensor container;
c) on successful polling wirelessly transferring information from the test sensor container to the meter and;
d) calculating the characteristic of the analyte or indicator using said quantity and the information
and in which step (c) is complete before the start of step (d).

3. A system according to claim 2 in which step (c) is complete before the end of step (a).

4. A system according to claim 2 or 3 in which step (c) is complete before the start of step (a).

5. A system according to any one of claims 2 to 4 adapted to carry out the step of (e) comparing two quantities representative of the characteristic of the analyte or indicator and in which step (b) commences after the result of step (e) is known.

6. A system according to any one of claims 2 to 5 in which step (a) includes counting down a number of seconds and step (c) commences during the count down.

7. A system according to claim 6 in which step (b) commences during the count down.

8. A system according to any one of claims 2 to 7 in which step (c) commences once a trigger level of a first predetermined quantity is reached.

9. A system according to claim 8 in which step (b) commences once a trigger level of a first predetermined quantity is reached.

10. A system according to claim 8 or 9 in which the first predetermined quantity is a first working electrode current.

11. A system according to claim 8, 9 or 10 in which a count down commences once the trigger level is reached.

12. A system according to any one of claims 2 to 11 in which step (c) commences once a trigger level of a second predetermined quantity is reached.

13. A system according to claim 12 in which step (b) commences once trigger level of a second predetermined quantity is reached.

14. A system according to any one of claims 2 to 13 in which second predetermined quantity is a second working electrode current.

15. A system according to claim 12, 13 and 14 in which the count down commences once the trigger level is reached.

16. A system according to any one of claims 2 to 15 in which step (b) commences once an initial threshold current is reached.

17. A system according to claim 14 in which the initial threshold is ≤ about 100nA.

18. A system according to any one of claims 2 to 17 comprising a step (f) displaying an "Apply Sample" request and one of step (b) and step (c) commences during step (f).

19. A system according to any one of claims 2 to 18 comprising step (f) displaying an "Apply Sample" request and one of step (b), step (c) is complete before step (f) commences.

20. A system according to any one of claims 2 to 19 comprising step (g) performing meter start up routine and one of step (b) and step (c) commences during step (g).

21. A system according to any one of claims 2 to 20 comprising step (g) performing meter start up routine and one of step (b) and step (c) is completed before the end of step (g).

22. A system according to any preceding claim in which the clip is adapted to releasibly receive one or both of the meter and test sensor container.
